# EUROPEAN PATENT SPECIFICATION

(11) **EP 3 753 564 B1**
(45) Date of publication and mention of the grant of the patent: **15.10.2025**
(21) Application number: 18906500.6
(22) Date of filing: 14.12.2018
(51) Int. Cl.: A61K 31/365, A61P 37/06

(54) **(5R)-5-HYDROXYTRIPTOLIDE FOR USE IN TREATMENT AND/OR PREVENTION OF IMMUNE DISORDERS IN AIDS**
(5R)-5-HYDROXYTRIPTOLID ZUR VERWENDUNG IN THERAPIE UND PRÄVENTION VON IMMUNERKRANKUNGEN BEI AIDS
(5R)-5-HYDROXYTRIPTOLIDE POUR UTILISATION POUR TRAITEMENT ET PRÉVENTION DES TROUBLES IMMUNITAIRES DANS SIDA

(30) Priority: 13.02.2018 CN 201810149149
(43) Date of publication of application: 23.12.2020
(73) Proprietor: Peking Union Medical College Hospital, Beijing 100000 (CN); Shanghai Pharmaceuticals Holding Co., Ltd., Shanghai 201203 (CN)
(72) Inventor: LI, Taisheng, Shanghai 201203 (CN); LIU, Yanjun, Shanghai 201203 (CN)
(74) Representative: Gulde & Partner
(86) International application number: PCT/CN2018/121201
(87) International publication number: WO 2019/157854

(56) References cited:
- CN-A- 1 511 838
- CN-A- 103 083 334
- ZHOU, R: "Inhibition of inducible nitric-oxide synthase expression by (5R)-5-hydroxytriptolide in interferon-gamma- and bacterial lipopolysaccha- ride-stimulated macrophages", JOURNAL OF PHARMACOLOGY AND EXPERIMENTAL THERAPEUTICS, vol. 316, no. 1, 31 December 2006 (2006-12-31), pages 121 - 128, XP055631810, ISSN: 0022-3565, DOI: 10.1124/jpet.105.093179

## Description

### Technical Field

The present disclosure pertains to the field of chemical pharmacy, and relates to a use of (5R)-5-hydroxytriptolide in treatment and/or prevention of abnormal immune activation in AIDS or incomplete immune reconstitution associated with AIDS.

### Background Art

*Tripterygium Wilfordii Hook f* (Lei Gong Teng) is a kind of vine plant of Celastraceae family, and mainly grows under humid environment near the mountain forest in the Yangtze River basin and southwest area of China. Its major chemical components include diterpenes, triterpenes, sesquiterpenes, alkaloids and the like. The pharmacological study in the last twenty years demonstrated that the extract of *Tripterygium Wilfordii Hook f* has anti-inflammatory, immunosuppressive, antifertility, anti-tumor and antibacterial activities. Especially in respect of immunosuppressive function, triptolide in *Tripterygium Wilfordii Hook f* was found to have significant biological activity. However, the clinical uses of triptolide are limited by its toxicity and side effects. The inventors of patent CN1223595C disclosed a series of novel triptolide derivatives through chemical modification on structure of this compound after studying the structure-activity relationship of triptolide thoroughly, which can be used as immunosuppressant for prevention and therapy against autoimmune diseases, infectious diseases and the like. Especially, it was demonstrated that (5R)-5-hydroxyltriptolide (LLDT-8, referred to as T8, having a structure as shown below), had anti-inflammatory and immunosuppressive activity with high potency but low toxicity and a good safety index, when evaluated in many *in vitro* assays and *in vivo* animal models.

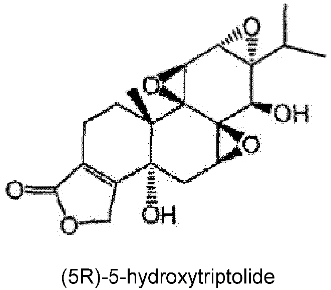

AIDS (Acquired Immune Deficiency Syndrome) is an infectious disease caused by human immunodeficiency virus (HIV) infection, which is characterized by HIV specifically attacking helper T lymphocytes (CD4⁺), causing the progressive malfunction of human immune system, and eventually causing various opportunistic infections and corresponding tumors. The highly active antiretroviral therapy (HAART) used since the 1990s can significantly reduce the plasma HIV load, and can effectively increase the CD4⁺ T-cells count, enabling patients to develop immune reconstitution, thereby reducing the occurrence of AIDS-related diseases and fatality rate. However, the latest research believes that HIV infection is a chronic inflammatory disease. The persistent activation of the immune system is the main immunopathological change occurring in the entire process of HIV infection, including the reduction of CD4⁺ T-cells caused by HIV infection, the formation and long-term maintenance of virus reservoirs, and the reconstruction obstacles. Especially, the complications and higher mortality of patients who have been successfully treated with long-term antiviral therapy are also considered to be directly or indirectly related to abnormal immune activation. Immune reconstitution refers to the process of recovery of immune system function in patients suffering from AIDS or bone marrow transplant or other patients with severe immunosuppression. When obstacles occur in the immune reconstitution process, it is called incomplete immune reconstitution. The incomplete immune reconstitution mainly occurs in AIDS patients, and these patients are also called INRs (immunological non-responders). For AIDS patients, INRs refer to patients whose viral load is below the lower limit of detection while CD4⁺ T-cells count does not recover (less than 250/µL) after receiving 2 years of formal antiretroviral therapy. Such patients are prone to a variety of opportunistic infections, tumors and the like, and have a high mortality rate. Abnormal immune activation refers to a long-term abnormally increased activation state of the immune system. Based on the fact that abnormal immune activation plays an important role in the onset of AIDS, the effect of antiviral therapy, and the occurrence of complications, in recent years, immune activation has been studied as a potential therapeutic target for AIDS. At present, there are no effective drugs in China and abroad for the treatment or prevention of abnormal immune activation or incomplete immune reconstitution.
In CN 103 083 334 A it is disclosed the use of triptolide in treatment of incompetent immune reconstitution of AIDS patients. Further, CN 1 511 838 A discloses a pharmaceutical composition prepared from triptolide derivatives, wherein said derivatives include (5R)-5-hydroxytriptolide.

### Content of the present invention

The present invention is directed to subject matter as defined in the claims. The technical problem to be solved in the present disclosure is to address the problem that there is no medicament for abnormal immune activation in AIDS and incomplete immune reconstitution associated with AIDS in the prior art, by providing a use of (5*R*)-5-hydroxytriptolide in treatment and/or prevention of abnormal immune activation in AIDS or incomplete immune reconstitution associated with AIDS.

The immune activation caused by HIV is different from the body's immune response and immune activation after viral infection in the common sense. Current research shows that its main mechanisms include:
1. Direct pathway: HIV gene products (such as gp120) directly activate lymphocytes and macrophages;
2. Indirect pathway: During HIV infection, because of the state of body's immune deficiency caused by HIV infection, some persistently infected pathogens in the body such as cytomegalovirus (CMV) and Epstein-Barr virus will undergo reactivation and replication, thereby causing immune activation;
3. Translocation of intestinal flora: The loss of a large number of CD4⁺ T-cells in the mucosal lymphatic tissue will cause the destruction of the immune components in the intestinal mucosal barrier, which will lead to the translocation of intestinal flora, thereby causing immune activation.
4. Decrease of regulatory T-cells (Treg): Treg plays an important role in maintaining the homeostasis of the body's immune system, and serve a role in down-regulating the immune activation state. Its activation and transformation are closely related to immune activation and immune reconstitution. Studies have shown that CD4⁺CD25⁺Treg in the HIV-infected long-term non-progressors is significantly higher than typical progressors, and the proportion of Treg is negatively correlated with the activated CD8⁺ T-cell subsets.
5. CD4⁺ T-cells pyroptosis: A study published by Gilad Doitsh et al. in Nature in 2014 showed that the vast majority (95%) of CD4⁺ T-cells death caused by HIV was accompanied by the release of inflammatory factors and intracellular contents such as IL-1β, and further induced caspase-1 mediated cell pyroptosis and promoted the accumulation, infection and pyroptosis of uninfected CD4⁺ T-cells in the body, thereby forming a vicious circle "pyroptosis-inducing the aggregation of uninfected CD4⁺ T-cells-infecting new CD4⁺ T-cells-new round of pyroptosis". In 2015, in a study published on *Cell Reports* by Galloway et al., a series of experiments proved that the direct contact (formation of synaptic connections) between infected CD4⁺ T-cells and uninfected CD4⁺ T-cells was a necessary condition to cause virus transmission and cell pyroptosis, and cell-free HIV virions, even with a high load, could not directly cause cell pyroptosis.

Persistent immune activation can have a series of serious consequences, including persistent HIV replication and immune reconstitution disorders, especially the increase in the mortality rate of AIDS patients, which cannot be completely eliminated even if the patients are subjected to a long-term effective antiviral therapy. The consequences mainly include the following several aspects:
1. Virus replication: The direct result of T-cell activation is the production of intracellular NF-κB, which increases the transcription of viral genes integrated into the host gene and produces new virus particles, thereby falling into a vicious circle.
2. Apoptosis: For activated T-cells, immune activation means that they will undergo differentiation, transformation and even apoptosis. Apoptosis related to immune activation is also considered to be an important reason for the decrease of peripheral CD4⁺ T-cells.
3. Immune senescence and exhaustion: In the adaptive immune response, naive T-cells are activated after contacting with antigen presenting cells, thereby proliferate and differentiate into effector T-cells and memory T-cells. However, persistent immune activation caused by persistent antigen stimulation or other mechanisms can cause effector T-cells to lose their function. This phenomenon is called "immune exhaustion". In addition, the thymus of the infected person also shrank to a degree disproportionate to their age, which means that the T-cells developed in the thymus cannot replenish the T-cells consumed in the periphery in time. Immune activation can also lead to the decline of bone marrow hematopoietic function, lymphatic tissue fibrosis, and the like.
4. Immune reconstitution obstacles: In immune non-responders, although antiviral therapy is effective and plasma virus replication is basically inhibited, there are still residual viruses, which may come from virus reservoirs (mainly memory T-cells) and sustainably low levels of virus replication. These residual viruses and their low-level replication are related to persistent immune activation and incomplete immune reconstitution.
5. Other abnormalities related to inflammation such as osteoporosis, atherosclerosis, neurocognitive degeneration, aging, and the like, are now also considered to be related to immune activation, leading to a significant increase in patient mortality.

Abnormal immune activation and inflammation caused by HIV infection is an important pathogenesis of non-AIDS-related diseases and incomplete immune reconstitution. Inhibiting excessive immune activation and inflammation may promote the recovery of CD4⁺ T lymphocytes in AIDS patients and reduce the occurrence of non-AIDS-related diseases. However, excessive inhibition may lead to aggravation of incomplete immune reconstitution, so it is important to find a drug that can inhibit abnormal immune activation without aggravating incomplete immune reconstitution. In terms of a drug, in addition to the therapeutic effect, the medication safety thereof also needs to be considered. The present inventors have inventively studied and found that (5R)-5-hydroxytriptolide (T8) inhibits excessive immune activation and inflammation in AIDS patients, and discovered a novel medicament for the treatment of abnormal immune activation or incomplete immune reconstruction in AIDS patients and for the treatment of non-AIDS related diseases.

In addition, the activation of the NF-κB pathway is essential for HIV replication and potential reactivation, and the NF-κB pathway controls the expression of multiple inflammatory factors in the cells.

The present disclosure discovers that (5R)-5-hydroxytriptolide (T8) can inhibit cellular immune activation by inhibiting NF-κB activity, and (5R)-5-hydroxytriptolide (T8) inhibits CD4⁺ T-cells subset activation and proliferation.

The present disclosure solves the above-mentioned technical problems through the following technical solutions:
The present disclosure provides a use of (5*R*)-5-hydroxytriptolide in the manufacture of a medicament for treatment and/or prevention of abnormal immune activation in AIDS or incomplete immune reconstitution associated with abnormal immune activation in AIDS. Wherein, the abnormal immune activation in AIDS is related to the incomplete immune reconstitution of AIDS (LIU Yu-chao; LI Tai-sheng, Immune Activation and Incomplete Immune Reconstitution in Chronic Human Immunodeficiency Virus Infected Patients, Medical Journal of Peking Union Medical College Hospita, Vol. 8, No. 4-5, p100-104, 2017).

In the present disclosure, the abnormal immune activation in AIDS is preferably NF-κB-related abnormal immune activation in AIDS.

In the present disclosure, the abnormal immune activation in AIDS is preferably chronic abnormal immune activation in AIDS.

In the present disclosure, the form of the medicament is not particularly limited, which can be solid tablet, liquid, gel, semi-liquid or aerosol form. Solid tablet is preferalbly.

In the present disclosure, the triptolide derivative is preferably one of the active ingredients in the medicament or the only active ingredient in the medicament.

The present disclosure also provides a use of (5*R*)-5-hydroxytriptolide in the manufacture of NF-κB signaling pathway inhibitor. The (5*R*)-5-hydroxytriptolide is preferably one of the active ingredients in the medicament or the only active ingredient in the medicament.

The present disclosure also provides a use of (5*R*)-5-hydroxytriptolide in the manufacture of T-lymphocyte activity inhibitor. The (5*R*)-5-hydroxytriptolide is preferably one of the active ingredients in the medicament or the only active ingredient in the medicament.

In the present disclosure, the above preferred conditions can be combined arbitrarily to obtain preferred embodiments of the present disclosure.

The reagents and raw materials used in the present disclosure are all commercially available.

The positive and progressive effects of the present disclosure are:
The present disclosure discovers that (5*R*)-5-hydroxytriptolide (T8) has immunosuppressive activity with high potency but low cytotoxicity in abnormal immune activation in AIDS or incomplete immune reconstitution associated with abnormal immune activation in AIDS, and has a good safety index after assays with (5*R*)-5-hydroxytriptolide (T8).

### Brief description of the drawings

Figure 1 shows the inhibition results of NF-κB activity by (5*R*)-5-hydroxytriptolide.
Figure 2 shows the inhibition results of CD4⁺ T-cells proliferation by (5*R*)-5-hydroxytriptolide.
Figure 3 shows the inhibitory effect of (5*R*)-5-hydroxytriptolide on the activation of CD4⁺ T-cell subsets.
Figure 4 shows the cytotoxicity test of (5*R*)-5-hydroxytriptolide (T8) and the reference substance (triptolide).
Figure 5 shows the inhibitory effect test results of (5*R*)-5-hydroxy triptolide (T8) and the reference substance (triptolide) on the activity of NF-κB signaling pathway.

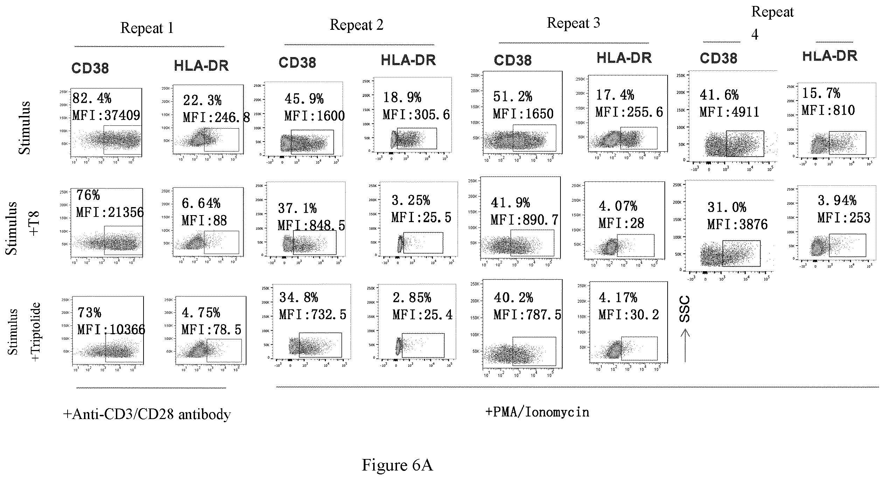

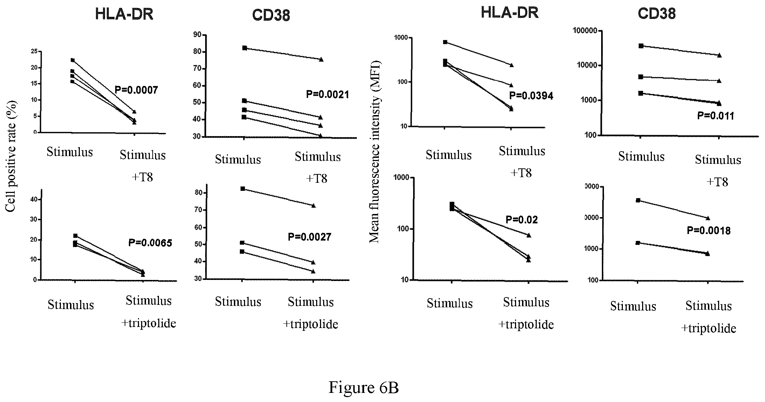

Figure 6 shows the inhibitory effect test results of (5*R*)-5-hydroxytriptolide (T8) and the reference substance (triptolide) on the immune activation of CD4⁺ T-cell subsets. CD4⁺ T-cells were isolated from the freshly isolated healthy human peripheral blood cell PBMCs using anti-CD4 antibody-coated magnetic beads through positive screening. The cells were stimulated with PMA (100 nM) and Ionomycin (1 µM) or anti-CD4 antibody-coated magnetic beads for 72 hours, and T8 or triptolide (100 ng/mL) was added at the same time. The cells were collected, stained with anti-CD4-PE antibody to gate CD4⁺ T-cells, and the expression of CD38 and HLA-DR on the cell surface was detected by flow cytometry. Figure 6A shows the results of flow cytometry in multiple completely independent repeated tests; Figure 6 B shows the statistical results of flow cytometry, counting the cell-positive rate and calculating the mean fluorescence intensity (MFI), respectively. Paired student t-test was used to analyze the significant differences.

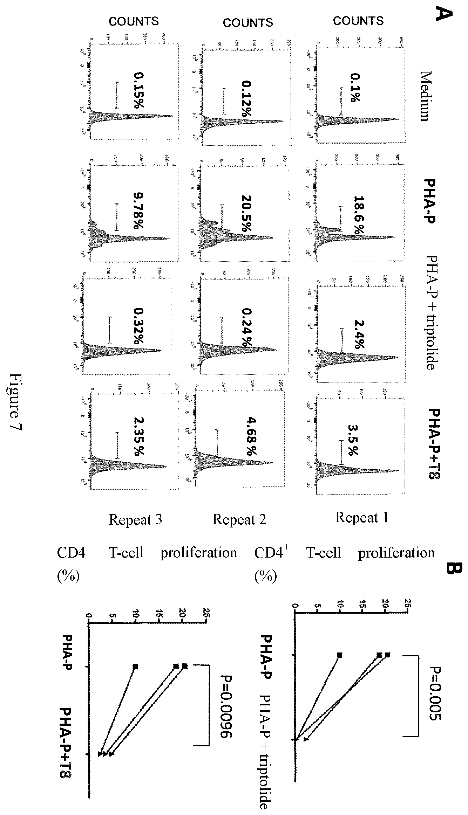

Figure 7 shows the inhibitory effects of T8 or triptolide on the proliferation of CD4⁺ T-cells. PBMCs were stained with CFSE; PHA-P (5 µg/mL) was added to induce the cell proliferation, and the test compound T8 or triptolide (100 ng/mL) was added at the same time; after 72 hours, the cells were collected and stained with anti-CD4-PE antibody. Flow cytometry was used to detect CFSE and analyze cell proliferation. (A) Test results of flow cytometry in three completely independent repeated tests; (B) Statistical results of flow cytometry. Paired student t-test was used to analyze the significant differences.

### Detailed description of the embodiment

The following examples further illustrate the present disclosure, but the present disclosure is not limited thereto. In the following examples, the experimental methods without specific conditions are carried out according to conventional methods and conditions, or according to the product specification.

### Example 1: the inhibitory effects of T8 on the activity of NF-κB

NF-κB driven luciferin expression plasmid (p3xB-luc, 100 ng) and internal reference plasmid pBgal (purchased from Bioonline: www.bioon.com.cn) (pSV-β-Galactosidase Control Vector, β-galactosidase expression plasmid, 5 ng) were transfected into HEK293T cells (purchased from ATCC). After 24 hours, the cells were treated with TNF-alpha (20 ng/mL) or untreated, and with or without the addition of T8 compound (100 nM). After another 24 hours, all cells were collected and lysed with reporter lysis buffer (purchased from Promega). Luciferase assay system (Promega) was used to detect luciferase activity, Beta-Glo Asaay system (Promega) was used to detect β-Galactosidase activity, and the inhibition effects of T8 on NF-κB activity was analyzed.

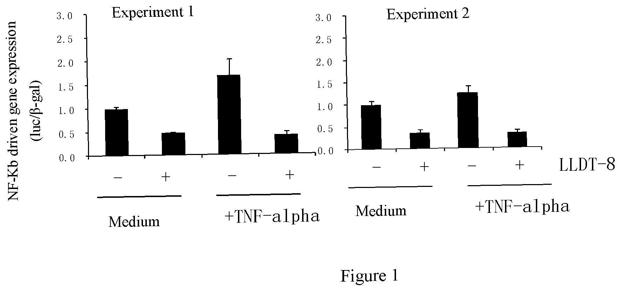

The test results are shown in Figure 1. The test results show (5*R*)-5-hydroxytriptolide has inhibitory effect on NF-κB activity. (5*R*)-5-hydroxytriptolide can not only effectively inhibit the background NF-κB activity (untreated with TNF-alpha), but also effectively inhibit NF-κB activity stimulated by TNF-alpha.

### Example 2: the inhibitory effects of T8 on the proliferation of CD4⁺ T-cells

Healthy human peripheral blood mononuclear cells (PBMC) (purchased from ATCC) were cultured in RPMI-1640/10% FBS serum medium containing IL-2 (20IU), and then stained with CFSE (carboxyfluorescein diacetate succinimidy ester) (10 nM) at 37 °C for 10 minutes, washed twice with the above medium, followed by addition of PHA-P (phytohemagglutinin P) (5 µg/mL) to stimulate cell proliferation with or without addition of T8 compound (100 nM). After 48 hours of culture, the cells were collected, stained with anti-human CD4 flow cytometry antibody (purchased from Invitrogen) (4 °C, 30 min), and CD4⁺ T-cells were gated by flow cytometry to analyze cell proliferation by detect the CFSE staining intensity.

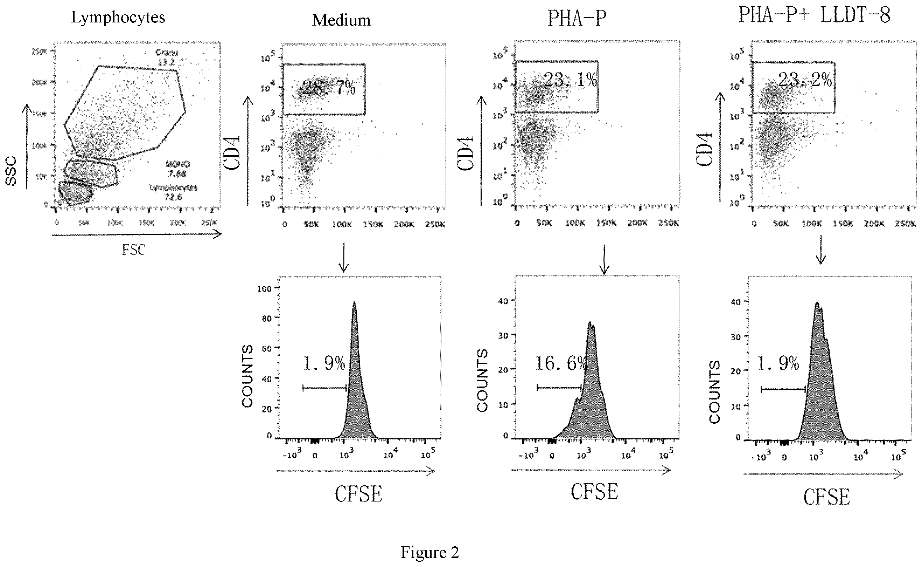

The test results are shown in Figure 2. The test results show that (5*R*)-5-hydroxytriptolide inhibits the proliferation of CD4⁺ T cells stimulated by PHA (cell proliferation is reduced from 16.6% to background proliferation 1.9%).

### Example 3: the inhibitory effect of T8 on the activation of CD4⁺ T-cell subsets

Healthy human PBMC (1 × 10⁷ cells) were stimulated with PMA (propylene glycol monomethyl ether acetate) (20 nM) and Ionomycin (1µM) at 37 °C with or without the addition of T8 compound (100 nM). After 48 hours, the cells were stained with anti-human CD4, CD38, HLA-DR flow cytometry antibody (purchased from Invitrogen), and the CD4⁺ T-cells were gated by flow cytometry to detect the expression of CD38, HLA-DR molecules on the cell surface.

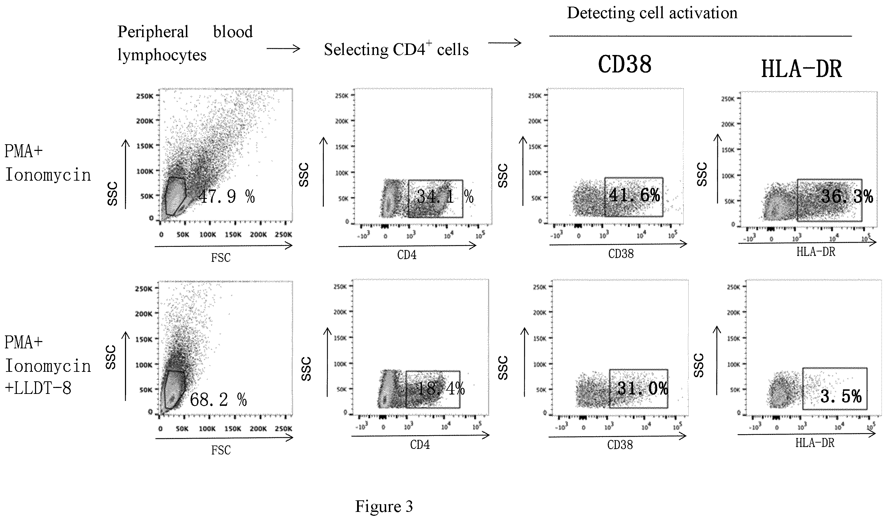

The test results are shown in Figure 3. The test results show that (5*R*)-5-hydroxytriptolide inhibits the activation of the cells stimulated by PMA+Ionomycin (CD38CD4/CD4 decreased from 41.6% to 31%; HLA-DRCD4/CD4 decreased from 36.3% to 3.5%).

### Comparative example 1: the cytotoxicity tests of (5R)-5-hydroxytriptolide (T8) and the reference substance (i.e., triptolide)

### 1. Experimental materials

### 1) Compounds to be tested

(5*R*)-5-hydroxytriptolide (T8) and the reference substance (triptolide), provided by Shanghai Pharmaceuticals Holding Co., LTD.; white powder, purity >99%, stored at 4 °C for later use;
Preparation methods: DMSO was used to prepare mother liquor, and medium (RPMI1640, Gibco) was used to prepare the working solution. The final concentration of DMSO during cell culture was <0.02%, which had no effect on cell growth.

### 2) Cells and reagents

HEK293T cells were cultured in DMEM complete medium containing 10% fetal bovine serum, 100 U/mL penicillin and 100 µg/mL streptomycin;
DMEM medium and fetal bovine serum were purchased from Gibco; Penicillin and streptomycin were purchased from Invitrogen;
MTT [3-(4,5-dimethylthiahiazol-2-yl)-2,5-diphenyltetrazolium bromide], SDS (Sodium dodecyl sulfate) and DMF (*N,N*-dimethylformamide) were purchased from Sigma;
MTT (Formazan) solution (100 mL): 10 g of SDS (Sodium dodecyl sulfate), 50 mL of DMF (*N,N*-dimethylformamide) and 50 mL of H₂O were mixed, heated, stirred evenly, and stored at 4 °C.

### 2. Experimental method

MTT colorimetric method was used to detect the cytotoxicity. HEK293T cells were seeded into 96-well plates (1 × 10⁴/100 µL/well), 100 µL of each concentration of the compounds (compound concentration refers to the figures) was added in triplicate. Control wells without compound were setting at the same time. The cells were incubated in a 37 °C, 5% CO₂ cell incubator for 72 hours. 100 µL of the supernatant was aspirated from each well and discarded, followed by addition of 20 µL of MTT solution (5 mg/mL) into the wells. The cells were incubated in a 37 °C, 5% CO₂ cell incubator for 4 hours, followed by addition of 100 µL of Formazan solution to each well and incubation overnight in the incubator until all Formazan was found to be dissolved under a common optical microscope. The absorbance was measured at 595 nm. The CC₅₀ value (50% Cytotoxic Concentration) was calculated, *i.e.,* the concentration of the compound required for producing toxicity on 50% of HEK293T cells.

### 3. Experimental results

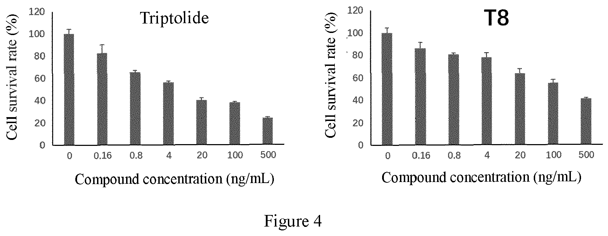

HEK293T cells were used to detect the cytotoxicity of the two compounds. It is found that the CC₅₀ (50% Cytotoxic Concentration) value of (5*R*)-5-hydroxytriptolide (T8) on HEK293T cells is about 435 ng/mL, while the CC₅₀ value of the reference substance (triptolide) on HEK293T cells is about 17 ng/mL, indicating that T8 has lower cytotoxicity compared with triptolide (see Figure 4 for details).

### Comparative example 2: the inhibitory effects of (5R)-5-hydroxytriptolide (T8) and the reference substance (triptolide) on the activity of NF-κB signaling pathway.

### 1. Experimental materials

### 1) The compounds to be tested were the same as above.

### 2) Cells and reagents

HEK293T cells were cultured in DMEM complete medium containing 10% fetal bovine serum, 100 U/mL penicillin and 100 µg/mL streptomycin.

DMEM medium, fetal bovine serum and OPTI-MEM were purchased from Gibco; Penicillin and streptomycin were purchased from Invitrogen; TNF-α was purchased from R&D; Lipofectamine 2000 Transfection Reagent was purchased from Life Technologies; Luciferase assay system and Glo Asaay system were purchased from Promega.

The pSV-β-Galactosidase Control Vector plasmid was from Promega (CATALOG # E1081).

### 2. Experimental method

The NF-κB reporter gene plasmid p3κB-luc was transfected into HEK293T cells for expression, and pSV-β-Galactosidase Control Vector was co-transfected as transfection internal reference. The activation of NF-κB was induced by TNF-α treatment, T8 and triptolide were added respectively. The expression of NF-κB reporter gene was indicated by detecting luciferase activity, and the effect of T8 and triptolide on NF-κB activation induced by TNF-α was evaluated.

Cell transfection: HEK293T cells were transfected with lipofectamine 2000 Transfection Reagent according to the reagent instructions. 100 ng of plasmid was transfected with 1 µL of transfection reagent into each well of a 24-well cell cultureplate. The plasmid and the transfection reagent were diluted with 50 µL of OPTI-MEM medium, respectively and then mixed together. The mixture was allowed to stand at room temperature for 5 minutes, and then added into the cells in each well. The resulting mixture was mixed gently, placed back into the 37 °C incubator, and the cells were cultured for a specified time.

Luciferase activity detection: NF-κB reporter gene plasmid p3κB-luc plasmid (100 ng), internal reference plasmid pSV-β-Galactosidase Control Vector (5 ng) were transfected into HEK293T cells. After 24 hours, TNF-α (20 ng/mL) was added (or not added) to stimulate the cells for 24 hours, and then the cells were collected and lysed with reporter lysis buffer. Luciferase assay system was used to detect luciferase activity, and Beta-Glo Asaay system was used to detect β-Galactosidase activity.

### 3. Experimental results

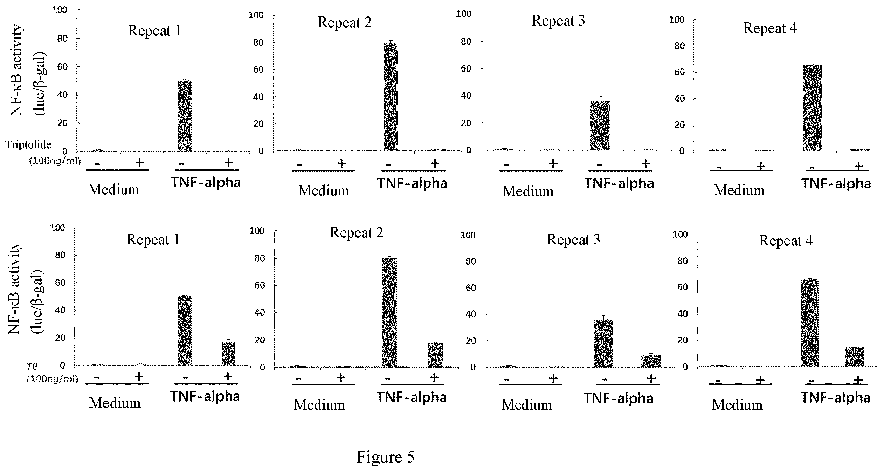

According to Figure 5, it can be seen that both (5*R*)-5-hydroxytriptolide (T8) and the reference substance (triptolide) can effectively inhibit the activity of the background NF-κB signaling pathway, and the activities of the two compounds are equivalent. The significant difference analysis in the inhibitory effects of the two compounds on the background NF-kB activity shows P=0.1, indicating no significant difference. (5*R*)-5-hydroxytriptolide (T8) and the reference substance (triptolide) can effectively inhibit the activity of the NF-kB signal pathway activated by TNF-α, and the activities of the two compounds are equivalent. The significant difference analysis in the inhibitory effects of the two compounds on the activity of NF-kB activated by TNF-α shows P=0.056, indicating no significant difference.

### Comparative example 3: the inhibitory effects of (5R)-5-hydroxytriptolide (T8) and the reference substance (triptolide) on immune cell activation

### 1. Experimental materials

### 1) The compounds to be tested were the same as above.

### 2) Cells and reagents:

Peripheral blood mononuclear cells (PBMCs) were purchased from Shanghai Changhai Hospital, and the cells were cultured in RPMI-1640 complete medium containing 10% fetal bovine serum, 100 U/mL penicillin, 100 µg/mL streptomycin, and 20 IU/mL IL-2 (R&D).

RPMI-1640 medium and fetal bovine serum were purchased from Gibco; penicillin and streptomycin were purchased from Invitrogen; IL-2 was purchased from R&D; phorbol ester (Phorbol 12-Myristate 13-Acetate, PMA) and Ionomycin were purchased from Sigma; anti-CD3/CD28 antibody-coated microbeads and CD4 antibody-coated microbeads were purchased from MACS; flow cytometry antibodies CD3, CD4, CD38, and HLA-DR were purchased from eBioscience.

### 2. Experimental method:

Freshly isolated healthy human PBMC (3 × 10⁷ cells) were cultured in cell culture medium containing IL-2 (20 IU/mL), stimulated with PMA (100 nM) and Ionomycin (1 µM) for 72 hours, and the compound to be tested (100 ng/mL) was added at the same time; the cells were washed twice with FACS buffer, followed by addition of anti CD3-PerCP, CD4-PE, CD38-PE-Cy7, HLA-DR-APC antibodies, stained for 30 minutes at 4 °C; the cells were washed twice with FACS buffer, and the expression of the above molecules was detected by FACS.

CD4⁺ T-cells were isolated from freshly isolated healthy human PBMCs with anti-CD4 antibody-coated magnetic beads through positive screening and then cultured in a medium containing IL-2 (20 IU/mL). The cell activation was stimulated with anti-CD3/CD28 antibody-coated magnetic beads for 72 hours, and the compound to be tested was added at the same time. The cells were collected, washed twice with FACS buffer, followed by addition of anti CD3-PerCP, CD4-PE, CD38-PE-Cy7, HLA-DR-APC antibodies, stained at 4 °C for 30 minutes; the cells were washed twice with FACS buffer and collected, and the expression of the above molecules was detected by flow cytometry.

### 3. Experimental results

By detecting the expression of cell activation indicators CD38 and HLA-DR, it is proved that (5*R*)-5-hydroxytriptolide (T8) and the reference substance (triptolide) can significantly inhibit the immune activation of CD4⁺ T-cell subsets by PMA+Ionomycin or anti-CD3/CD28 antibody.

(5*R*)-5-Hydroxytriptolide (T8) and the reference substance (triptolide) have comparable effects in inhibiting the immune activation of CD4⁺ T-cell subsets (see Figure 6 for details).

### Comparative example 4

### 1. Experimental materials:

### 1) The compounds to be tested is the same as above.

### 2) Cells and reagents:

Peripheral blood mononuclear cells (PBMCs) were purchased from Shanghai Changhai Hospital, and the cells were cultured in RPMI-1640 complete medium containing 10% fetal bovine serum, 100 U/mL penicillin, 100 µg/mL streptomycin, and 20 IU/mL IL-2 (R&D). The cells were cultured for 72 hours in a complete medium containing 5 µg/mL phytohemagglutinin (Phytohemagglutinin-P, PHA-P) to stimulated the proliferation of CD4⁺ T-cells.

RPMI-1640 medium and fetal bovine serum were purchased from Gibco; Penicillin and streptomycin were purchased from Invitrogen; IL-2 was purchased from R&D; PHA-P was purchased from Sigma; CFSE dye was purchased from abcam; Anti-CD4-PE antibody was purchased from eBioscience.

### 2. Experimental method:

Freshly isolated healthy human PBMC (1 × 10⁷ cells) were cultured in cell culture medium containing IL-2 (20 IU/mL), and were then stained with CFSE (10 nM) at 37 °C for 10 minutes. The cells were washed with the medium twice, PHA-P (5 µg/mL) was added, and the test compound T8 or triptolide (100 ng/mL each) was added at the same time. After 72 hours at 37 °C, the cells were collected, stained with anti-CD4-PE antibody at 4 °C for 30 minutes, and washed with FACS buffer twice. The cell proliferation was analyzed by flow cytometry.

### 3. Experimental results

According to Figure 7, it can be seen that (5*R*)-5-hydroxy triptolide (T8) and the reference substance (triptolide) can significantly inhibit the proliferation of CD4⁺ T-cells in the healthy human peripheral blood and have a comparable effect.

Although the specific embodiments of the present disclosure are described above, those technician in this filed should understand that these are intended to be illustration, and various changes or modifications can be made to these embodiments. Therefore. the protection scope of the present disclosure is defined by the appended claims.

## Claims

1. (5*R*)-5-hydroxytriptolide for use in treatment and/or prevention of abnormal immune activation in AIDS or incomplete immune reconstitution associated with abnormal immune activation in AIDS.

2. The (5*R*)-5-hydroxytriptolide for use as defined in claim 1, wherein the abnormal immune activation in AIDS is NF-xB-related abnormal immune activation in AIDS.

3. The (5*R*)-5-hydroxytriptolide for use as defined in claim 1 or 2, wherein the abnormal immune activation in AIDS is chronic abnormal immune activation in AIDS.

4. The (5*R*)-5-hydroxytriptolide for use as defined in claim 1, wherein the (5*R*)-5-hydroxytriptolide is comprised in a medicament which is in the form of solid tablet.

5. The (5*R*)-5-hydroxytriptolide for use as defined in claims 1 to 4, wherein the (5*R*)-5-hydroxytriptolide is presented as one of the active ingredients in a medicament or as the only active ingredient in a medicament.

## Patentansprüche

1. (5*R*)-5-Hydroxytriptolid zur Verwendung in der Therapie und/oder Prävention von abnormaler Immunaktivierung bei AIDS oder von mit abnormaler Immunaktivierung bei AIDS assoziierter unvollständiger Immunrekonstitution.

2. (5*R*)-5-Hydroxytriptolid zur Verwendung nach Anspruch 1, wobei die abnormale Immunaktivierung bei AIDS eine NF-κB-bezogene abnormale Immunaktivierung bei AIDS ist.

3. (5*R*)-5-Hydroxytriptolid zur Verwendung nach Anspruch 1 oder 2, wobei die abnormale Immunaktivierung bei AIDS eine chronische abnormale Immunaktivierung bei AIDS ist.

4. (5*R*)-5-Hydroxytriptolid zur Verwendung nach Anspruch 1, wobei das (5*R*)-5-Hydroxytriptolid in einem Medikament enthalten ist, welches in Form einer festen Tablette ist.

5. (5*R*)-5-Hydroxytriptolid zur Verwendung nach den Ansprüchen 1 bis 4, wobei das (5*R*)-5-Hydroxytriptolid als eines der Wirkstoffe in einem Medikament oder als einziger Wirkstoff in einem Medikament vorliegt.

## Revendications

1. (5*R*)-5-hydroxytriptolide pour utilisation dans le traitement et/ou la prévention d'une activation immunitaire anormale dans le SIDA ou d'une reconstitution immunitaire incomplète associée à une activation immunitaire anormale dans le SIDA.

2. (5*R*)-5-hydroxytriptolide pour utilisation tel que défini dans la revendication 1, dans lequel l'activation immunitaire anormale dans le SIDA est une activation immunitaire anormale liée à NF-κB dans le SIDA.

3. (5*R*)-5-hydroxytriptolide pour utilisation tel que défini dans la revendication 1 ou 2, dans lequel l'activation immunitaire anormale dans le SIDA est une activation immunitaire anormale chronique dans le SIDA.

4. (5*R*)-5-hydroxytriptolide pour utilisation tel que défini dans la revendication 1, dans lequel le (5*R*)-5-hydroxytriptolide est compris dans un médicament qui est sous la forme d'un comprimé solide.

5. (5*R*)-5-hydroxytriptolide pour utilisation tel que défini dans les revendications 1 à 4, dans lequel le (5*R*)-5-hydroxytriptolide est présenté comme l'un des principes actifs dans un médicament ou comme le seul principe actif dans un médicament.
